# EUROPEAN PATENT SPECIFICATION

(11) **EP 2 424 540 B1**
(45) Date of publication and mention of the grant of the patent: **23.04.2014**
(21) Application number: 10770239.1
(22) Date of filing: 28.04.2010
(51) Int. Cl.: A61K 31/553, A61P 13/10

(54) **COMPOSITIONS AND METHODS FOR TREATING AND PREVENTING OVERACTIVE BLADDER AND CONDITIONS ASSOCIATED THEREWITH**
ZUSAMMENSETZUNGEN UND VERFAHREN ZUR BEHANDLUNG UND PRÄVENTION VON BLASENHYPERAKTIVITÄT UND DAMIT VERBUNDENEN LEIDEN
COMPOSITIONS ET MÉTHODES DE TRAITEMENT ET DE PRÉVENTION DE L'INCONTINENCE URINAIRE ET DES PATHOLOGIES ASSOCIÉES

(30) Priority: 28.04.2009 US 173398 P
(43) Date of publication of application: 07.03.2012
(73) Proprietor: Optmed, Inc., New York, NY 10017 (US)
(72) Inventor: BROOKOFF, Daniel, Memphis TN 38104 (US)
(74) Representative: Comoglio, Elena
(86) International application number: PCT/US2010/032703
(87) International publication number: WO 2010/126963

(56) References cited:
- US-A1- 2006 030 622
- US-A1- 2008 070 904
- KONTANI H ET AL: "A METHOD FOR PRODUCING OVERACTIVE BLADDER IN THE RAT AND INVESTIGATION OF THE EFFECTS OF GABAergic RECEPTOR AGONISTS AND GLUTAMATERGIC RECEPTOR ANTAGONISTS ON THE CYSTOMETROGRAM", JOURNAL OF UROLOGY, LIPPINCOTT WILLIAMS & WILKINS, BALTIMORE, MD, US, vol. 173, no. 5, 1 May 2005 (2005-05-01), pages 1805-1811, XP027855200, ISSN: 0022-5347 [retrieved on 2005-05-01]
- FUJIKAWA KEITA ET AL: "Human atrial natriuretic peptide is a useful criterion in treatment of nocturia", SCANDINAVIAN JOURNAL OF UROLOGY AND NEPHROLOGY, vol. 35, no. 4, September 2001 (2001-09), pages 310-313, XP009163513, ISSN: 0036-5599
- WELLS W G ET AL: "Use of in-office anesthesia during non-surgical radiofrequency collagen denaturation for stress urinary incontinence", CURRENT MEDICAL RESEARCH AND OPINION 200706 GB LNKD- DOI:10.1185/030079907X188161, vol. 23, no. 6, June 2007 (2007-06), pages 1279-1284, XP9163530, ISSN: 0300-7995

## Description

### FIELD OF THE INVENTION

The present invention provides a new therapeutic application of diazepam.

### BACKGROUND OF THE INVENTION

Overactive bladder syndrome ("OAB") is a condition that encompasses urinary urge incontinence, urgency and frequency. Urinary incontinence is the inadvertent leakage of urine caused by the inability of the bladder to retain urine as a consequence of urge (urge incontinence) or stress (stress incontinence). It is estimated that 17 million Americans suffer with OAB.

Normal bladder function, both storage and micturition (or voiding), is the result of intricate coordination between the detrusor muscles of the bladder, the striated muscles of the external urethra, the smooth muscle of the internal urethra and the neuronal inhibitory system. This inhibitory mechanism is believed to involve the inhibition of parasympathetic activity or the increase in sympathetic tone to produce detrusor relaxation which allows filling to occur. During filling the outlet neck of the bladder and urethra are contracted preventing leakage. Micturition is characterized by a relaxation of the outlet neck of the bladder and of the urethra followed by contraction of the detrusor muscle. When the bladder is empty the detrusor muscle relaxes and the outlet neck and urethra contract sealing off the bladder to maintain continence.

Bladder wall contraction appears to be influenced by parasympathetic innervation while the internal sphincter of the urethra/bladder neck is principally under sympathetic control. When the parasympathetic system is abnormally activated, acetylcholine binds to muscarinic receptors on bladder smooth muscle cause detrusor contraction mediating micturition. Acetylcholine is the principal neurotransmitter in detrusor muscle. Antimuscarinic drugs are often used to inhibit these contractions.

Many of the symptoms of OAB are attributable to urodynamically-demonstrable detrusor overactivity. Three urodynamic patterns of urethral pressure variation are described in patients with detrusor instability: 1) Rapid pressure variation ("RPV"), associated with onset at low bladder volumes and with detrusor instability; 2) gradual pressure variation ("GPV"), which begins at high bladder volumes; and 3) stress-induced transient urethral relaxation which is not associated with any specific pattern of urethral pressure variation or detrusor pressure change. Patients with a combination of RPV and detrusor instability reveal a statistically significant increase in the frequency of urethral relaxation as the primary precipitant of unstable detrusor contractions. RPV is the pattern most commonly associated with detrusor instability.

"Classic" OAB appears to be ignited by a sensory disorder. Alteration of the sensitivity of afferent activation or loss of control over transmitter release could lead to sensory- or motor-activated incontinence, respectively. It has been hypothesized that a layer of myofibroblasts immediately below the basal lamina of the urothelium acts as a "variable gain" regulator of the sensory process between ATP release and afferent excitation. These fibroblasts are functionally connected to form an electrical syncitium, make close contact with nerves and respond by generating electrical impulses and transient increases in intracellular Ca⁺⁺ when exposed to ATP.

GABA-ergic/glycinergic inhibitory transmission has been implicated in the central control of sphincter reflexes in the urinary bladder, which to some degree is mediated by spinal interneuron activity. For example, infusion of fluid through the urethra facilitates bladder contractions and alterations on primitive bladder-to-urethra and urethra-to-bladder reflex mechanisms may contribute to neurogenic bladder dysfunction.

In addition to dysfunctions caused by lesions to the bladder or urethra, damage to the brain itself can induce bladder overactivity by reducing supra-pontine inhibition. Direct damage to axonal pathways in the spinal cord can also lead to the emergence of primitive spinal bladder reflexes triggered by C-fiber bladder afferent neurons.

Patients with detrusor instability have demonstrated anatomic as well as neurochemical abnormalities such as decreased urethral longitudinal smooth muscle thickness, decreased total urethral diameter and decreased total urethral circumference compared to subjects with normal urodynamic testing. This suggests an anatomical basis for physiologic findings in patients with "urethrogenic" detrusor instability. These patients are defined urodynamically as demonstrating an un-inhibitable elevation in intravesical pressure during bladder filling. Smooth muscles from unstable bladders often show enhanced spontaneous contractile activity. This allows spontaneous electrical activity to spread and initiate synchronous contractions throughout the detrusor muscle. Unstable bladders often show patchy denervation of the muscle bundles.

Urge incontinence is the most problematic feature of OAB and may be particularly devastating if the amount of urine lost is large. Being incontinent of urine and wetting in public is certainly the most embarrassing symptom for the majority of people who suffer with OAB. Patients with OAB are likely to report a specific type of urinary urgency which is associated with sudden onset and accompanied by a compelling fear of urinary leakage.

The effects of incontinence can be particularly devastating for women. Decreased quality of life correlates with urinary frequency, nocturia (waking at night one or more times to void) and incontinence pad use. OAB causes sleep disturbance to the point of significantly diminishing the quality of life in 10% of women affected. OAB has a higher impact in the quality of life of younger women, whose concerns include loss of bodily control, wetness, fear of smell and the need to stay close to home. OAB is also a common cause of sexual dysfunction in millions of women, disrupting intimacy and kindling dyspareunia.

Incidence of OAB increases with age, and urinary incontinence has been reported to represent one of the top four important health-related quality of life problems affecting senior citizens. In a large survey of community-dwelling elderly adults, over 40% of those with OAB did not consult a physician. Of those who had consulted a physician, the vast majority had not received treatment at the time of the survey and reported that their OAB symptoms had a negative impact upon their quality of life. Co-morbidities associated with - OAB included risk of falls and fractures among the elderly patients due to rushing to the toilet in addition to more urinary tract infections, skin infections, sleep disturbance and depression than age-matched controls. Thus, people with OAB have a significantly poorer quality of life than age-matched controls, and lifestyle adaptations to OAB include: limiting daily travel, reducing fluid intake, avoiding sexual intimacy, wearing incontinence pads and carrying extra clothes.

Accordingly, restoration of continence is one of the primary objectives of a treatment of OAB, though other symptoms such as frequency, urgency and nocturia are also targets of therapy.

Consumer satisfaction with the current medical treatments of OAB is very low. A large survey showed that the majority of the patients with OAB reported that they were not satisfied with anti-muscarinic treatments. Those with stress or mixed incontinence were more satisfied than those with urge incontinence; nonetheless, almost half observed no change in outcome from their most helpful treatment and very few reported being cured. It has been shown that over half of all OAB sufferers would welcome some new form of treatment but that they are reluctant to seek new therapies. In a survey of people who had undergone medical treatment for OAB, half reported that they had discontinued the medication at some point for inadequate efficacy, adverse events or intolerability and cost.

Antimuscarininc agents are, by far, the most commonly used medications for the treatment of OAB and include the highly lipophilic tertiary amines oxybutinin, tolterodine, solifenacin and darifenacin, and the quaternary amine trospium. The newest treatment approved by FDA is the second-generation antimuscarinic fesoteridine.

Oxybutinin is available in immediate- and extended-release oral forms and a transdermal patch. Oxybutinin is highly associated with adverse cognitive effects and effects on sleep architecture and quality. One of its metabolites, the N-desmethyl-oxybutinin, is probably responsible for the side effects of dry mouth which can be minimalized to some extent by the use of the transdermal route.

Tolterodine is available as immediate-release and extended-release oral preparations. It is less lipophilic than oxybutinin, but the extended release form is comparable in efficacy to oxybutinin and reportedly causes reduced dry mouth. Tolterodine has also been associated with adverse cognitive effects and effects on sleep architecture and quality.

Solifenacin has a higher affinity for M-1 and M-3 receptors than for M-2 receptors giving it the highest degree of bladder selectivity. Darifenacin has a reduced affinity for M-1 receptors which may reduce its cognitive side effects to some extent.

Trospium has been used in Europe for over 20 years and was approved by the FDA in 2004. It is the only quaternary amine antimuscarininc drug available. Because of its hydrophilicity, it is minimally metabolized (60% is excreted completely active) which leads to fewer drug-drug interactions. Trospium, however, is still associated with antimuscarinic side effects.

The newest approved oral treatment, fesoterodine, does not offer much better tolerability, as it is still associated with constipation, dry mouth and sometimes urinary tract infection.

The results with anticholinergic treatments have been quite variable. The extended release preparations of either oxybutinin or tolterodine did not appear to confer much advantage except for the possibility of reduced dry mouth. Even with the availability of extended release formulations, up to 80% of patients being treated for OAB discontinue their medications within 6 months due to side effects.

There is no single superior antimuscarininc agent and the individual response to each agent is highly variable. All of the above antimuscarinic medications are relatively contraindicated in narrow-angle glaucoma, bladder outflow obstruction, decreased gastric motility or reduced renal or hepatic function. The spectrum of anticholinergic central nervous system ("CNS") side effects includes drowsiness, hallucinations, severe cognitive impairment and even coma. The following table lists side effects associated with the various antimuscarinic drugs as reported in the literature:

**Table 1**

| Antimuscarinic Agent | Dose / day | Adverse Events |
|---|---|---|
| oxybutynin chloride | 5-30 mg oral | dry mouth |
| | | constipation |
| | | somnolence |
| | | headache |
| | | diarrhea |
| | | nausea |
| | | asthenia |
| | | pain |
| | | dyspepsia |
| | | dizziness |
| | | blurred vision |
| | | rhinitis |
| | | urinary tract infection |
| | 3.9 mg transdermal | pruritis |
| | | erythema |
| | | dry mouth |
| tolterodine tartrate | 4 mg | dry mouth |
| | | headache |
| | | constipation |
| darifenacin | 7.5 mg | dry mouth |
| | | constipation |
| | 15 mg | dry mouth |
| | | constipation |
| | | dyspepsia |
| solifenacin succinate | 5 mg | dry mouth |
| | | constipation |
| | | small bowel pseudo-obstruction |
| | 10 mg | dry mouth |
| | | constipation |
| trospium chloride | 20 mg x 2 | dry mouth |
| | | constipation |
| fesoterodine | 4 mg or 8 mg QD | dry mouth |
| | | constipation |
| | | urinary tract infection |

The potential CNS adverse side effects of the anticholinergics have to be weighed against the severity of OAB symptoms, especially in the treatment of elderly patients. Elderly patients are more prone to these side effects and are more likely to be taking prescriptions and over-the-counter medications with anticholinergic properties (for example antihistamines) which, when combined with antimuscarinic for the treatment of OAB may result in a substantial anticholinergic load and put them at greater risk for significant cognitive side effects.

kontani et al (173, 1805-11, May 2005. The journal of Urology), described the intraperitoneal administration of diazepam on a rat model of bladder overactivity.

In addition to the pharmacological agents discussed above, there are alternative non-pharmaceutical modalities for treatment of urge incontinence, including, for example, behavioral therapies. While it has been found that pelvic exercise can reduce urinary incontinence by 50-70%, the effectiveness of these techniques appears to be limited to stress incontinence.

Accordingly, there is great need for more effective compositions and methods for the treatment and/or prevention of OAB and associated conditions.

### SUMMARY OF THE INVENTION

This invention relates to the surprising discovery that local delivery of an effective amount of a pharmaceutical composition comprising diazepam is capable of treating and/or preventing OAB and conditions associated therewith, without the side effects often associated with previous treatments.

In one embodiment, this invention relates to diazepam for use for preventing or treating overactive bladder or urinary incontinence by administering diazepam intravaginally to a female mammal for whom such prevention or treatment is needed or desirable.

In another embodiment, this invention relates to diazepam for use for preventing or treating overactive bladder or urinary incontinence by administering diazepam intraurethrally to a mammal for whom such prevention or treatment is needed or desirable.

### DETAILED DESCRIPTION OF THE INVENTION

As used herein, a "pharmaceutical composition" refers to any combination of two or more components. It may be in the form of, for example, suppositories, ointments, solutions, gels, sprays, creams, suspensions, liquids, powders, pastes, aqueous, non-aqueous or any combination thereof.

The term "diazepam" is used herein to refer to 7-chloro-1,3dihydro-1 -methyl-5-phenyl-2*H*-1,4-benzodiazepin-2-one and all pharmaceutically-acceptable forms.

By an "effective amount" is meant a nontoxic, but sufficient, amount of diazepam to treat or prevent incidence or severity of OAB. An effective amount of diazepam is preferably less than about 50mg. In certain embodiments an effective amount is from about 1mg to about 30mg. In other embodiments an effective amount is from about 5mg to about 20mg. In certain embodiments, an effective amount is less than about 5mg.

The present invention relates to the surprising discovery that the delivery of relatively high local concentrations of diazepam is surprisingly effective in treating and/or preventing the incidence and/or severity of OAB without triggering the side effects which commonly occur with previous treatments.

### Dosage Forms

Dosage forms for transmucosal vaginal or intraurethral administration of diazepam may include for example suppositories, ointments, solutions, gels, sprays, creams, suspensions, liquids, powders, pastes, aqueous, non-aqueous. The active ingredient may be mixed under sterile conditions with a pharmaceutically-acceptable carrier.

Suppositories consist of a combination of pharmacologically active component with a suppository base. Suitable suppository bases are, for example, natural or synthetic triglycerides or paraffin hydrocarbons. Solutions generally consist of homogenous mixtures of a pharmacologically active substance in a solvent. Gels generally comprise a liquid organic phase entrapped in a three-dimensionally cross-linked network. The liquid may be an organic solvent, a mineral oil or a vegetable oil. Sprays generally include a dynamic collection of liquid drops and the entrained surrounding gas. Suspensions generally include dispersions of solid particles in fluids. Pastes generally include suspensions of small particles dispersed in a background fluid comprising a fatty base (*e.g*., petroleum jelly) and generally 25% or more of solid substance (*e.g*., zinc oxide).

Ointments are semisolid preparations which are typically based on petrolatum or other petroleum derivatives. As with other carriers or vehicles, an ointment base should be inert, stable, nonirritating and nonsensitizing. Creams containing the selected active agent, are, as known in the art, viscous liquid or semisolid emulsions, either oil-in-water or water-in-oil. Cream bases are water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant.

Dosage forms according to the present invention may contain, in addition to diazepam, carriers or excipients, such as animal and vegetable fats, oils, waxes, paraffins, starch, tragacanth, cellulose derivatives, polyethylene glycols, propylene glycols, glycerine, silicones, bentonites, silicic acid, talc and zinc oxide, other synthetic solvents, or mixtures thereof.

Emulsifiers and surfactants used as excipients in certain dosage forms according to the present invention may include: nonionic ethoxylated and nonethoxylated surfactants, abietic acid, almond oil polyethylene glycol, beeswax, butylglucoside caprate, C₁₈-C₃₆ acid glycol ester, C₉-C₁₅ alkyl phosphate, caprylic/capric triglyceride polyethylene glycol-4 esters, ceteareth-7, cetyl alcohol, cetyl phosphate, corn oil polyethylene glycol esters, dextrin laurate, dilaureth-7 citrate, dimyristyl phosphate, glycereth-17 cocoate, glyceryl erucate, glyceryl laurate, hydrogenated castor oil polyethylene glycol esters, isosteareth-11 carboxylic acid, lecithin, lysolecithin, nonoxynol-9, octyldodeceth-20, palm glyceride, polyethylene glycol diisostearate, polyethylene glycol stearamine, poloxamines, potassium linoleate, raffinose myristate, sodium caproyl lactylate, sodium caprylate, sodium cocoate, sodium isostearate, sodium tocopheryl phosphate, steareths, and trideceths, aluminum starch octenylsuccinate, ammonium hydroxide, amphoteric-9, beeswax, synthetic beeswax, carbomer 934, carbomer 934P, carbomer 940, ceteareth-20, ceteareth-30, cetearyl alcohol, ceteth 20, cetyl alcohol, cholesterol, cyclomethicone, diglycerides, dimethicone (e.g., dimethicone 350), disodium monooleamidosulfosuccinate, NF emulsifying wax, fatty acid pentaerythritol ester, glycerides, glyceryl monooleate, glyceryl monostearate, lanolin, lanolin alcohol, hydrogenated lanolin, magnesium stearate, mineral oil, monoglycerides, polyethylene glycol, PEG 100 stearate, polyethylene glycol 6000 distearate, polyethylene glycol 1000 monocetyl ether, polyethylene glycol monostearate, polyethylene glycol 400 monostearate, polyoxyethylene glycol fatty alcohol ethers, polyoxyl 20 cetostearyl ether, polyoxyl 40 stearate, polysorbate 20, polysorbate 40, polysorbate 60, polysorbate 80, polysorbates, PPG-26 oleate, propylene glycol stearate, quaternium-15, simethicone, sodium laureth sulfate, sodium lauryl sulfate, sorbitan esters, sorbitan monolaurate, sorbitan monooleate, sorbitan monopalmitate, sorbitan monostearate, sorbitan palmitate, sorbitan sesquioleate, steareth-2, steareth-100, stearic acid, stearyl alcohol, triethanolamine and trolamine. Other surfactants and emulsifiers may be used, as will be appreciated by one of ordinary skill in the art.

Compositions according to the present invention also may include a wide range of other optional excipients including, for example, antifoaming agents; buffers, neutralizing agents and agents to adjust pH; coloring agents and decoloring agents; emollients and emulsion stabilizers; humectants; odorants; preservatives, antioxidants, and chemical stabilizers; solvents; and stiffening and suspending agents. Exemplary antifoaming agents include cyclomethicone, dimethicone (e.g., dimethicone 350) and simethicone. Exemplary buffers, neutralizing agents and agents to adjust pH include ammonium hydroxide, citric acid, diisopropanolamine, hydrochloric acid, lactic acid, monobasic sodium phosphate, sodium citrate, sodium hydroxide, sodium phosphate, triethanolamine, and trolamine. Exemplary emollients include caprylic/capric triglyerides, castor oil, ceteareth-20, ceteareth-30, cetearyl alcohol, ceteth 20, cetostearyl alcohol, cetyl alcohol, cetyl stearyl alcohol, cocoa butter, diisopropyl adipate, glycerin, gyceryl monoolcate, glyceryl monostearate, glyceryl stearate, isopropyl myristate, isopropyl palmitate, lanolin, lanolin alcohol, hydrogenated lanolin, liquid paraffins, linoleic acid, mineral oil, oleic acid, white petrolatum, polyethylene glycol, polyoxyethylene glycol fatty alcohol ethers, polyoxypropylene 15-stearyl ether, propylene glycol stearate, squalane, steareth-2 or -100, stearic acid, stearyl alcohol and urea.

Exemplary emulsion stabilizers and viscosity builders include carbomer 934, carbomer 934P, carbomer 940, cetearyl alcohol, cetostearyl alcohol, cetyl alcohol, cetyl stearyl alcohol, dextrin, diglycerides, disodium edetate, edetate disodium, glycerides, glyceryl monostearate, glyceryl stearate, hydroxypropyl cellulose, monoglycerides, plasticized hydrocarbon gel, polyethylene glycol 300, polyethylene glycol 400, polyethylene glycol 1450, polyethylene glycol 8000, polyethylene glycols, propylene glycol stearate and stearyl alcohol. Exemplary humectants include glycerine, propylene glycol, sorbitol and urea. Exemplary odorants include hypoallergenic perfume, menthol. Exemplary preservatives, antioxidants, and chemical stabilizers include alcohol, benzyl alcohol, butylated hydroxyanisole, butylated hydroxytoluene, butylparaben, calcium acetate, caster oil, chlorocresol, 4-chloro-m-cresol, citric acid, disodium edetate, Dowicil 200 (Dow), edetate disodium, ethoxylated alcohol, ethyl alcohol, glycerin, Glydant Plus (Lonza), 1,2,6-hexanetriol, Kathon CG (Rohm & Haas), Liquid Germall Plus (ISP Sutton Labs), Liquipar (ISP Sutton Labs), methylparaben, parabens, potassium sorbate, propyl gallate, propylene glycol, propylparaben, sodium bisulfite, sodium citrate, sodium metabisulfite, sorbic acid, tannic acid, triglycerides of saturated fatty acids, Ucarcide (Union Carbide), and zinc stearate. Exemplary solvents include alcohol, castor oil, diisopropyl adipate, ethoxylated alcohol, ethyl alcohol, fatty alcohol citrate, glycerin, 1,2,6-hexanetriol, hexylene glycol, isopropyl alcohol, isopropyl myristate, isopropyl palmitate, mineral oil, phosphoric acid, polyethylene glycol 300, polyethylene glycol 400, polyethylene glycol 1450, polyethylene glycol 8000, polyethylene glycol 1000 monocetyl ether, polyethylene glycol monostearate, polyethylene glycol 400 monostearate, polyethylene glycols, polyoxyl 20 cetostearyl ether, polyoxypropylene 15-stearyl ether, polysorbate 20, polysorbate 40, polysorbate 60, polysorbate 80, polysorbates, propylene carbonate, propylene glycol, purified water, and SD alcohol 40, triglycerides of saturated fatty acids.

Thickening, stiffening and suspending agents suitable for inclusion in compositions according to the present invention may include, for example, agents commonly used in skin care preparations. More specifically, such excipients include acrylamides copolymer, agarose, amylopectin, calcium alginate, calcium carboxymethyl cellulose, carbomer, carboxymethyl chitin, cellulose gum, dextrin, gelatin, hydrogenated tallow, hydroxyethylcellulose, hydroxypropylcellulose, hydroxpropyl starch, magnesium alginate, methylcellulose, microcrystalline cellulose, pectin, various polyethylene glycol's, polyacrylic acid, polymethacrylic acid, polyvinyl alcohol, various polypropylene glycols, sodium acrylates copolymer, sodium carrageenan, xanthan gum, yeast beta-glucan, aluminum stearate, beeswax, synthetic beeswax, carbomer 934, carbomer 934P, carbomer 940, cetostearyl alcohol, cetyl alcohol, cetyl esters wax, dextrin, glyceryl monostearate, hydroxypropyl cellulose, kaolin, paraffin, petrolatum, polyethylene, propylene glycol stearate, starch, stearyl alcohol, wax, white wax, xanthan gum, and bentonite.

In one embodiment of the present invention diazepam powder may be suspended in propylene glycol to form a smooth paste. Xanthan gum may be optionally included to increase adherence. In certain embodiments the formulation is preferably a low volume viscous formulation which may be applied with an applicator device to deposit the dosage form in the vaginal wall adjacent to the cervix. Other excipients may include Methocel E4M 3%, plasticized ointment, carafate and petrolatum base. These excipients alone or in combination may increase bulk and/or slow the release of diazepam.

In another embodiment of the present invention pharmaceutical formulations include diazepam in a matrix-based gel. Such matrix-based gels may include carbomer and/or hydroxycellulose, polycarbophils, propylene glycol, glycerin and water. In another embodiment suitable formulations may be in the form of vesicle/inclusion-based creams which may include lipids and/or beta cyclodextrin in a hydrophilic cream base.

Such formulations optionally may include preservatives such as sorbic acid and/or benzyl alcohol. In one embodiment sorbic acid may be used as a preservative in matrix-based gels. In other embodiment benzyl alcohol may be used as a preservative in a vesicle/inclusion-based cream. In certain embodiments other preservatives may be used in addition to or instead of sorbic acid and/or benzyl alcohol.

In certain embodiments pharmaceutical formulations also may include various organic solvents to increase the solubility of diazepam. Other FDA-approved excipients may be included as well.

In certain embodiments the formulations are adjusted to a pH level between about 3 and 6, more preferably between about 4 and 5.

Representative formulations may include diazepam at concentrations from about 1 to about 20 mg/ml. Specific concentrations within this range may be prepared. For example, in certain embodiments diazepam concentrations may be formulated to include 2, 5 or 10 mg/ml.

Such formulations allow for extended duration of therapeutic effect while minimizing serum levels of diazepam or its metabolites following vaginal application.

Each composition disclosed herein may be packaged in a container appropriate in for its viscosity and intended use by the patient. For example, a cream may simply be stored in a non-deformable bottle, or in a squeeze container, such as a tube, or a lidded jar. Such formulations may be stored at room temperature and are preferably protected from light. Care should be taken for storing such formulations since diazepam is incompatible with certain types of plastics. -

### Local Delivery

If diazepam is useful in influencing the spinal or supraspinal micturition reflex in humans, certainly its sedative and cognitive effects would limit its clinical use to treat OAB at the doses that would be required. Accordingly, formulations according to the present invention comprise diazepam administered via the intravaginal route in women or the intraurethral route in women and men suffering from OAB. This results in decreased bladder spasms without the sedative side-effects associated with the oral formulation of diazepam.

Cognitive impairment is usually not associated with plasma levels below 100 ng/ml of plasma. Following the administration of an effective amount of diazepam-containing compositions, the maximum combined plasma level of diazepam and of its primary active metabolite, desmethyldiazepam, preferably will not exceed 100 ng/ml of plasma. In certain embodiments this maximum combined plasma level preferably will not exceed 50 ng/ml and even more preferably will not exceed 25 ng/ml of plasma. Local delivery of diazepam for the treatment and/or prevention of OAB and its symptoms thus eliminates, or greatly reduces, cognitive impairment associated with traditional oral or parenteral use.

It is understood by those skilled in the art that the dosage amount will vary with the severity of OAB, the age, size and condition of the patient, and like factors known in the medical art. In general, a suitable dose will be that amount of the compound which is the lowest dose effective to reduce the incidence or severity of OAB without toxicity. However, a precise dosage may be determined by an attending physician within the scope of sound medical judgment.

### EXAMPLES

The examples which follow illustrate exemplary embodiments of the compositions and methods of the present

### Example 1 Representative Compositions

Representative compositions according to the present invention are shown below in Tables 1A and 1B.

**Table 2A - Vaginal Suppositories**

| Diazepam | Glycerine | Beeswax |
|---|---|---|
| 5 mg | ✔ | |
| 5mg | | ✔ |
| 10 mg | ✔ | |
| 10 mg | | ✔ |
| 15 mg | ✔ | |
| 15 mg | | ✔ |
| 20 mg | ✔ | |
| 20 mg | | ✔ |

As shown in Table 1A, representative vaginal suppositories may contain 5 to 20 mg of diazepam in combination with glycerin and/or beeswax. Other excipients may be included as well. Such compositions can be delivered locally to the bladder to treat, prevent or significantly reduce the severity of OAB.

**Table 2B - Gels**

| Diazepam | Methylcellulose | Hydroxyethylcellulose |
|---|---|---|
| 5 mg | ✔ | |
| 5mg | | ✔ |
| 10 mg | ✔ | |
| 10 mg | | ✔ |
| 15 mg | ✔ | |
| 15 mg | | ✔ |
| 20 mg | ✔ | |
| 20 mg | | ✔ |

As shown in Table 1B, representative gels which can be used for intraurethral administration may include from 5 to 20mg of diazepam in combination with methylcellulose and/or hydroxyethylcellulose. Other excipients may be included as well. Such compositions can be delivered locally to the bladder to treat, prevent or significantly reduce the severity of OAB.

### Example 2

Effect of diazepam administered intravenously and intravaginally on cystometries in anesthetized rats.

Methods: Female rats were anesthetized with urethane (1.3 g/kg intraperitoneally) given as a divided dose of 80% initially and 20% 15 minutes later. Body temperature was maintained at 37±2 oC throughout the experiment. A catheter was inserted into the bladder through the urethra. Then, the urethra was ligated at the level of the urinary meatus. After an abdominal incision, the ureters were also ligated. The jugular vein was catheterized to allow administration of test compounds. The bladder catheter was connected via a T-tube to a strain gauge to measure intravesicular pressure. Isovolumetric bladder contractions were induced by stepwise injections of physiological saline at room temperature (100 µl every 5 min) until stable rhythmic bladder contractions occurred. After stabilization and a control period of at least 30 min, characterized by stable and reproducible bladder contractions (used as basal values), test substance or vehicle was administered i.v. (as a bolus under a volume of 200 µl) or i.vag. (under a volume of 100 µl using a syringe). The drug effects were evaluated for 1 hour following the end of administration. Each animal received one dose of one test substance.

Results: In comparison with basal values, intravaginal delivery of diazepam resulted in significant reductions in micturition frequency and micturition amplitude (threshold for voiding) through 30 minutes post administration. These reductions were comparable to those achieved by intraveneous administration.

### Example 3

A female in her 50s had been suffering from symptoms consistent with OAB for several months. Following a confirmatory diagnosis of OAB, the subject was instructed to use vaginal suppositories containing 20mg of diazepam in a glycerine base three times a day. This treatment greatly reduced her symptoms of urgency, frequency and incontinence. No cognitive impairment was observed or reported. The subject continues to do well on a maintenance dose of 20mg once or twice a day.

### Example 4

A male in his 60s had been suffering from symptoms consistent with OAB for several months. A diagnosis of OAB was confirmed by an urologist. A nurse then administered intraurethrally to the subject 2 ml of a gel formulation containing 10mg/ml of diazepam in 2% methylcellulose, providing a total dose of 20mg of diazepam. This reduced OAB symptoms significantly without causing any observable cognitive impairment. This subject is able to treat himself at home using the same type of formulation and the same route of administration up to 3 times per day as needed.

### Example 5

A 19 year old male presented with urge incontinence following a head injury which resulted in ethmoid neuritis. His condition resulted in intractable nocturia and nighttime urinary incontinence. His symptoms were not relieved with high doses of oral diazepam but were well-controlled by intraurethral instillations of 2 ml of diazepam in a gel formulation containing 10 mg/ml of diazepam in hydroethylcellulose, providing a total of 20 mg of diazepam administered at bedtime. The subject did not suffer any adverse effects from these treatments.

### Example 6

An 85 year old female with advanced dementia suffered from OAB symptoms including nocturia and urge incontinence. Despite the use of incontinence pads, the subject would frequently arise throughout the night without requesting assistance to go the bathroom. This led to numerous falls. Treatment with oral antimuscarinic and oral diazepam led to cognitive impairments which increased the number of falls from bed. Intraurethral instillation of 1 ml of diazepam in a gel formulation containing 20 mg/ml of diazepam in 3% hydroxyethylcellulose three times per day reduced daytime urgency without causing cognitive impairment or impairing her ability to urinate during the day (albeit still using an incontinence pad). With these treatments, the subject was able to sleep in bed throughout the night without arising to urinate without the need for any restraining devices. The family noted a near-absence of nighttime falls once the intraurethral treatments had been started. She suffered no apparent adverse effects from these treatments.

## Claims

1. Diazepam for use for preventing or treating overactive bladder or urinary incontinence by intravaginal administration to a female mammal or intraurethral administration to a mammal of an effective amount of diazepam.

2. Diazepam for use according to claim 1 , wherein said mammal is human.

3. Diazepam for use according to claim 1 or 2, wherein following said intravaginal or intraurethral administration of diazepam combined plasma level of diazepam and desmethyldiazepam does not exceed about 100ng/ml.

4. Diazepam for use according to claim 3, wherein following said intravaginal or intraurethral administration of diazepam, the combined plasma level of diazepam and desmethyldiazepam does not exceed about 50ng/ml.

5. Diazepam for use according to claim 4, wherein following said intravaginal or intraurethral administration of diazepam, the combined plasma level of diazepam and desmethyldiazepam does not exceed about 25ng/ml.

6. Diazepam for use according to any of claims 1 to 5 , wherein the effective amount of diazepam is less than about 50mg.

7. Diazepam for use according to claim 6, wherein the effective amount of diazepam is from about 1mg to about 30mg.

8. Diazepam for use according to claim 7, wherein the effective amount of diazepam is from about 5mg to about 20mg.

9. Diazepam for use according to 6, wherein the effective amount of diazepam is less than about 5mg.

## Patentansprüche

1. Diazepam für die Verwendung zur Vorbeugung oder Behandlung von Blasenhyperaktivität oder Harninkontinenz durch intravaginale Verabreichung an ein weibliches Säugetier oder intraurethrale Verabreichung an ein Säugetier einer wirksamen Menge an Diazepam.

2. Diazepam für die Verwendung nach Anspruch 1, wobei das Säugetier menschlich ist.

3. Diazepam für die Verwendung nach Anspruch 1 oder 2, wobei in der Folge der intravaginalen oder intraurethralen Verabreichung von Diazepam der kombinierte Plasmaspiegel an Diazepam und Desmethyldiazepam etwa 100 ng/ml nicht übersteigt.

4. Diazepam für die Verwendung nach Anspruch 3, wobei in der Folge der intravaginalen oder intraurethralen Verabreichung von Diazepam der kombinierte Plasmaspiegel an Diazepam und Desmethyldiazepam etwa 50 ng/ml nicht übersteigt.

5. Diazepam für die Verwendung nach Anspruch 4, wobei in der Folge der intravaginalen oder intraurethralen Verabreichung von Diazepam der kombinierte Plasmaspiegel an Diazepam und Desmethyldiazepam etwa 25 ng/ml nicht übersteigt.

6. Diazepam für die Verwendung nach einem der Ansprüche 1 bis 5, wobei die wirksame Menge an Diazepam weniger als etwa 50 mg ist.

7. Diazepam für die Verwendung nach Anspruch 6, wobei die wirksame Menge an Diazepam von etwa 1 mg bis etwa 30 mg ist.

8. Diazepam für die Verwendung nach Anspruch 7, wobei die wirksame Menge an Diazepam von etwa 5 mg bis etwa 20 mg ist.

9. Diazepam für die Verwendung nach Anspruch 6, wobei die wirksame Menge an Diazepam weniger als etwa 5 mg ist.

## Revendications

1. Diazépam pour utilisation en vue de traiter ou prévenir une hyperactivité de la vessie ou une incontinence urinaire, par administration intra-vaginale chez un mammifère femelle, ou par administration intra-urétrale chez un mammifère, d'une quantité efficace de diazépam.

2. Diazépam pour utilisation conforme à la revendication 1, ledit mammifère étant un humain.

3. Diazépam pour utilisation conforme à la revendication 1 ou 2, dans laquelle, à la suite de ladite administration intra-vaginale ou intra-urétrale de diazépam, le niveau plasmatique combiné de diazépam et déméthyl-diazépam n'excède pas environ 100 ng/mL.

4. Diazépam pour utilisation conforme à la revendication 3, dans laquelle, à la suite de ladite administration intra-vaginale ou intra-urétrale de diazépam, le niveau plasmatique combiné de diazépam et déméthyl-diazépam n'excède pas environ 50 ng/mL.

5. Diazépam pour utilisation conforme à la revendication 4, dans laquelle, à la suite de ladite administration intra-vaginale ou intra-urétrale de diazépam, le niveau plasmatique combiné de diazépam et déméthyl-diazépam n'excède pas environ 25 ng/mL.

6. Diazépam pour utilisation conforme à l'une des revendications 1 à 5, dans laquelle la quantité efficace de diazépam vaut moins d'environ 50 mg.

7. Diazépam pour utilisation conforme à la revendication 6, dans laquelle la quantité efficace de diazépam vaut d'environ 1 mg à environ 30 mg.

8. Diazépam pour utilisation conforme à la revendication 7, dans laquelle la quantité efficace de diazépam vaut d'environ 5 mg à environ 20 mg.

9. Diazépam pour utilisation conforme à la revendication 6, dans laquelle la quantité efficace de diazépam vaut moins d'environ 5 mg.
